# EUROPÄISCHE PATENTSCHRIFT

(11) **EP 0 813 058 B1**
(45) Veröffentlichungstag und Bekanntmachung des Hinweises auf die Patenterteilung: **17.12.2003**
(21) Anmeldenummer: 97108108.8
(22) Anmeldetag: 15.05.1997
(51) Int. Cl.: G01N 27/416

(54) **MOS-Transistor für biotechnische Anwendungen**
MOS transistor for biotechnical applications
Transistor MOS pour des applications biotechniques

(30) Priorität: 12.06.1996 DE 19623517
(43) Veröffentlichungstag der Anmeldung: 17.12.1997
(73) Patentinhaber: Infineon Technologies AG, 81669 München (DE)
(72) Erfinder: vom Felde, Andreas, D-81669 München (DE); Bertagnolli, Emmerich, 80799 München (DE); Kerber, Martin, 81827 München (DE)
(74) Vertreter: Kindermann, Peter, Dipl.-Ing.

(56) Entgegenhaltungen:
- DE-A- 3 940 540
- FR-A- 2 724 489
- PATENT ABSTRACTS OF JAPAN vol. 9, no. 210 (P-383), 28.August 1985 & JP 60 073352 A (HITACHI)

## Beschreibung

Die Aufzeichnung der elektrischen Aktivität und der elektrischen Reizung von lebenden Neuronenzellen ist eine Vorbedingung zur Untersuchung der biologischen Mechanismen, insbesondere der Weiterleitung von Nervenimpulsen, der Detektion von Neuronen und der Lernfähigkeit von Nervengewebe. Derartige Erkenntnisse bilden die Grundlage für den zukünftigen Bau von neuronalen Biosensoren und die Realisierung von neuro-elektronischen Schaltungen. Die elektrische Ankopplung von lebenden Neuronzellen an MOS-Transistoren ist derzeit Gegenstand von Untersuchungen. Eine elektrisch leitende Verbindung zwischen einer Zellmembran und der Gate-Elektrode eines MOS-Transistors ist möglich, falls ein Transistor ohne Gate-Polysilizium und ohne Metallisierung verwendet wird. Ein.Problem bei der Verwendung von Halbleiterbauelementen für derartige Untersuchungen ist die mangelnde Resistenz vieler für diese Bauelemente verwendeter Materialien gegenüber den für die lebenden Zellen verwendeten Nährlösungen. Diese Nährlösungen bilden einen Elektrolyten, der viele Materialien elektrochemisch korrodiert.

Aufgabe der vorliegenden Erfindung ist es, einen MOS-Transistor anzugeben, der für biotechnische Anwendungen geeignet ist und der insbesondere elektrochemisch korrosionsfest ist.

Diese Aufgabe wird mit dem Transistor mit den Merkmalen des Anspruches 1 gelöst. Weitere Ausgestaltungen ergeben sich aus den abhängigen Ansprüchen.

Der erfindungsgemäße Transistor besitzt eine Gate-Elektrode, die elektrisch leitend mit einer Kontaktfläche oder Anschlußfläche (Pad) verbunden ist. Diese Kontaktfläche ist für die betreffende biotechnische Anwendung dimensioniert. Sie besitzt z. B. Abmessungen, die an die Größe einer biologischen Zelle, die mit dem Gate des Transistors leitend verbunden werden soll, angepaßt sind. Diese Kontaktfläche ist aus einem Material hergestellt, das elektrochemisch korrosionsfest ist, also weder von der Zellmembran noch von einer Nährlösung oder einem den Kontakt herstellenden Haftmittel angegriffen wird. Die restliche Oberfläche dieses Transistors ist mit einer Passivierungsschicht bedeckt, die im wesentlichen nur diese Kontaktfläche frei läßt und die gegenüber den Elektrolyten, denen der Transistor bei der Anwendung ausgesetzt wird, resistent ist. Für eine derartige passivierende Deckschicht kommt insbesondere eine dünne Abscheidung eines Nitrides in Frage. Eine weitere Eigenschaft des erfindungsgemäßen Transistors ist eine relativ flache Oberfläche, d. h. die Oberflächentopologie zumindest im Bereich um die Kontaktfläche besitzt Höhenunterschiede von höchstens 200 nm. Das erleichtert die Ankopplung von Zellen an die Kontaktfläche.

Bei besonders bevorzugten Ausführungsformen des erfindungsgemäßen Transistors ist das Verhältnis von Gateweite zu Gatelänge größer als 3. Die Gatelänge ist die Abmessung der Gateelektrode in der Richtung von Source nach Drain; die Gateweite ist die Abmessung der Gate-Elektrode im rechten Winkel zur Gatelänge in dem für die Funktionsweise des Transistors maßgeblichen Bereich um den Kanal. Mit dieser zusätzlichen Optimierung erreicht man eine möglichst große Steilheit der Kennlinien des Transistors. Auf diese Weise bietet der Transistor eine ausreichend hohe Empfindlichkeit, um auch bei spontaner Aktivität der zu untersuchenden Nervenzellen (Änderungen des intrazellulären Potentials von etwa 40 mV bis 60 mV) Änderungen des Gatepotentials von 40 mV in Änderungen des Drainstromes im Bereich von µA zu transformieren.

Bei einer weiteren besonders bevorzugten Ausführungsform ist das Verhältnis der Fläche (Flächeninhalt) der Kontaktfläche zu der Fläche der Gate-Elektrode (die Fläche des Gate-Steges in Aufsicht, die Gatelänge multipliziert mit der Gateweite) größer als 5. Damit wird erreicht, daß die Gate-Elektrode eine wesentlich kleinere Kapazität gegenüber dem Substrat besitzt als eine Zellmembran einer auf die Kontaktfläche aufgebrachten biologischen Zelle. Die Kapazitäten der Zellmembran und des Transistors bilden dann einen optimierten kapazitiven Spannungsteiler, der es erlaubt, ein Höchstmaß des Spannungshubs der Zellmembran auf das Gate im aktiven Transistorbereich zu übertragen. Diese Eigenschaft wird unterstützt durch eine Minimierung der Fläche der Gate-Elektrode (Gatebreite x Gatelänge), um die Kapazität der Gate-Elektrode zum Substrat möglichst klein zu halten. Für Zellmembranen geringer Abmessungen kann dann die Kontaktfläche ebenfalls klein gehalten werden.

Vorzugsweise werden die Gate-Elektrode, die Kontaktfläche und die elektrisch leitende Verbindung dazwischen aus Polysilizium im Rahmen eines CMOS-Prozesses hergestellt. Bei einem derartigen Prozeß ist es möglich, weitere Schaltungskomponenten für die Auswertung von Meßsignalen mit dem erfindungsgemäßen Transistor zusammen auf einem Substrat zu integrieren. Das Polysilizium wird z. B. mit einer Metallsilizierung versehen. Beim Aufbringen eines Metalles auf das Polysilizium wird das Metall teilweise in das Silizium einlegiert und bildet eine besondere Art chemischer Verbindung mit dem Silizium. Eine derartige im Prinzip bereits bekannte Silizierung, wie sie auch bei anderen Bauelementen hergestellt wird, kann bei dem erfindungsgemäßen Transistor vorzugsweise mit Titan realisiert werden. Neben Titan sind auch andere silizierfähige Metalle verwendbar, z. B. Tantal, Wolfram, Kobalt, Molybdän, Platin oder Palladium, die ebenfalls ausreichend korrosionsfest und für eine Verbindung mit biologischen Organismen geeignet sind.

Statt einer derartigen Silizierung oder ergänzend dazu kann eine vollständige (z. B. Mehrlagen-)Metallisierung vorhanden sein. Dafür kommt insbesondere Wolfram als Metall für die Oberfläche der Kontaktfläche, für die elektrisch leitende Verbindung zur Gate-Elektrode und für die Metallisierung der Gate-Elektrode in Frage. Mit einer derartigen Metallisierung ist insbesondere auch die elektrisch leitende Verbindung zu den Komponenten einer integrierten elektronischen Schaltung realisierbar. Bei einer derartigen Metallisierung sind die Leitungswiderstände wesentlich kleiner als bei den Siliziden. Außer Wolfram sind für diese Metallisierungen auch Titan, Tantal, Kobalt, Molybdän, Platin, Iridium, Palladium und TiN oder Kombinationen dieser Materialien verwendbar.

Die passivierende Deckschicht, die die Oberseite des Transistors mit Ausnahme der Kontaktfläche bedeckt, ist vorzugsweise eine dünne Nitridschicht (z. B. SiN), die vorzugsweise mittels CVD in einer Dicke von mindestens 50 nm und höchstens 250 nm aufgebracht wird. Auf diese Weise werden im Bereich der Kontaktfläche niedrige Verhältnisse von Tiefe zu Durchmesser der Kontaktstelle realisiert. Dieses sogenannte Aspektverhältnis der Kontaktstelle ist eine wichtige Größe, die die Stärke der Kopplung zwischen Zellmembran und Transistor bestimmt.

Bei Gateweiten im Sub-µm-Bereich und mit Abständen von Gate zu Gate von weniger als 10 µm lassen sich Arrays von erfindungsgemäßen Transistoren realisieren, mit denen eine hohe räumliche Auflösung erreichbar ist, so daß auch größere Nervenzellen höher entwickelter Organismen damit untersucht werden können.

Typische Abmessungen des erfindungsgemäßen Transistors beim derzeitigen Stand der Herstellungstechnologien sind z. B. eine Gatelänge von etwa 0,8 µm und eine Gateweite von 5,0 µm. Die Kontaktfläche kann z. B. 10 µm x 10 µm groß sein. Es sind aber auch Kontaktflächen realisierbar, die für Zellmembranen eines Durchmessers von 1 µm optimiert sind.

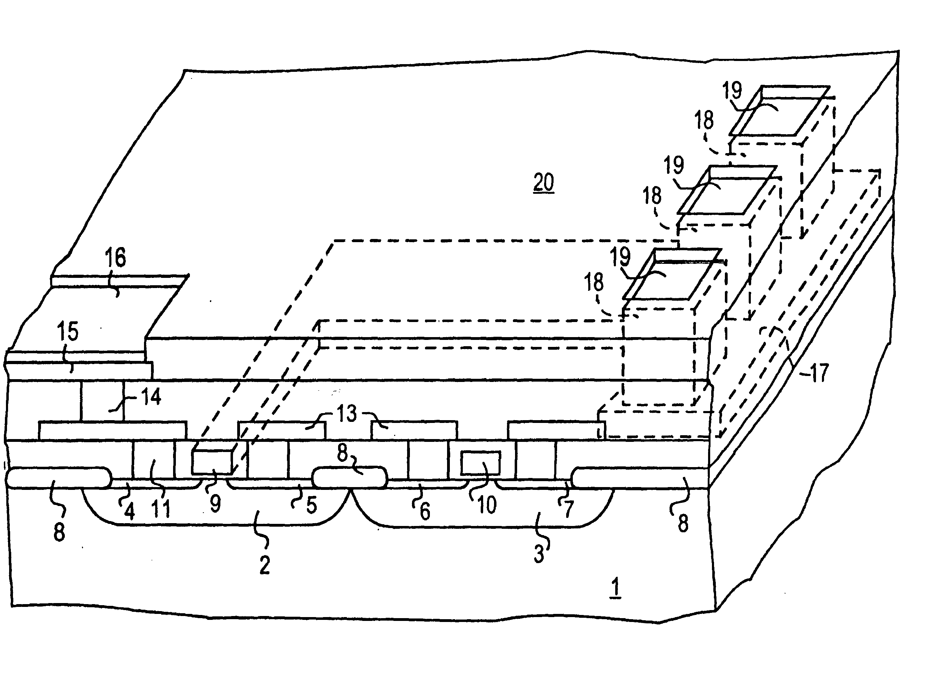

In der beigefügten Figur ist ein mögliches Ausführungsbeispiel für den erfindungsgemäßen Transistor dargestellt. In einem Substrat 1, das z. B. Silizium ist, befinden sich n-Wannen und/oder p-Wannen 2, 3, in denen dotierte Bereiche 4, 5, 6, 7 als Source- und Drain-Bereiche von MOSFETs vorhanden sind. Für den erfindungsgemäßen Transistor, der in der Figur links dargestellt ist, kann z. B. eine n-Wanne 2 vorgesehen sein. Ein weiterer dazu komplementärer MOSFET ist z. B. in einer p-Wanne 3 ausgebildet. Die Bereiche von Source 4 und Drain 5 des erfindungsgemäßen Transistors sind dann p-leitend, die Bereiche von Source 6 und Drain 7 des dazu komplementären Transistors sind dann n-leitend. Die eingezeichneten Transistoren sind nur als Beispiel zu verstehen; auf dem Substrat 1 kann eine grundsätzlich unbegrenzte Zahl von MOSFETs, die im Rahmen des CMOS-Verfahrens hergestellt werden, integriert sein.

Zwischen diesen Transistoren befinden sich isolierende Bereiche 8, die z. B. durch eine thermische Oxidation (LOCOS) hergestellt sind. Die Gate-Elektrode 9 ist auf dem Gate-Oxid im Bereich des Kanals des erfindungsgemäßen Transistors aufgebracht. Wie die gestrichelt eingezeichneten verdeckten Konturen zeigen, ist die Gate-Elektrode 9 seitlich zu einer größeren Anschlußfläche 17 geführt, auf der Blöcke oder Säulen 18 in der Art von Kontaktlochfüllungen aufgebracht sind. Die Gate-Elektrode 9 ist vorzugsweise Polysilizium. Eine entsprechende Gate-Elektrode 10, die aber z. B. über die vorgesehenen Metallisierungsebenen 13 mit einer auf dem Substrat 1 integrierten Schaltung verbunden sein kann, ist für den weiteren MOSFET vorgesehen. In einer ersten Dielektrikumschicht befinden sich vertikale leitende Verbindungen 11, 12, die z. B. als Kontaktlochfüllungen über den anzuschließenden Sourceund Drain-Bereichen hergestellt sind. Diese erste Dielektrikumschicht ist vorzugsweise wie üblich z. B. Borphosphorsilikatglas (BPSG). Über der Metallisierungsebene 13, die zu Anschlußkontakten oder Leiterbahnen strukturiert ist, können eine oder mehrere weitere Dielektrikumschichten (Zwischenoxid, IMOX) vorgesehen sein. Darin sind weitere vertikale elektrisch leitende Verbindungen 14 ausgebildet. Die eingezeichnete Verbindung 14 führt zu einer Anschlußfläche 15 auf der Oberseite, die mit einer korrosionsfesten Metallisierung 16 bedeckt ist. Die Oberseiten 19 der Blöcke oder Säulen 18 auf der Anschlußfläche 17 der Gate-Elektrode 9 bestehen ebenfalls aus korrosionsfestem Material. Die Säulen 18 können vollständig aus diesem korrosionsfesten Material bestehen und werden dann z. B. als Kontaktlochfüllungen hergestellt. Es genügt aber im Prinzip, wenn die nach außen freie Oberseite 19 elektrochemisch korrosionsfest ist. Die Oberseiten 19 dieser Säulen 18 sind dafür vorgesehen, daß Neuronzellen in einer Nährlösung darauf aufgebracht werden, und sind daher entsprechend dimensioniert. Die Anschlußfläche 15 mit der ebenfalls elektrochemisch korrosionsfesten Metallisierung 16 ist z. B. als Anschlußkontakt für eine Messung (Meßpad) vorgesehen. Die Oberseite 20 der Anordnung wird durch die passivierende Deckschicht gebildet, die vorzugsweise eine dünne Nitridschicht ist (CVD-Nitrid), die die Oberseiten 19, auf die die Neuronzellen aufgebracht werden sollen, wie oben beschrieben nur wenig überragt.

Die gesamte Oberseite der Gate-Elektrode 9, der Anschlußf läche 17 und der dazwischen angeordneten Verbindung kann mit einer Metallisierung versehen sein. Falls für diese Metallisierung ein gegen die Nährlösung der Neuronzellen beständiges Material verwendet wird, kann man auf die Säulen 18 verzichten und die Neuronzellen in einer entsprechenden Aussparung der Passivierung unmittelbar auf diese den Anschlußbereich 17 bedeckende Metallisierung aufbringen. Die Säulen 18 können eine geringere seitliche Abmessung besitzen als in der Figur eingezeichnet. Es genügt, wenn Kontaktflächen mit einer für die Größe der Neuronzellen ausreichenden Fläche und mit einer korrosionsfesten Oberfläche vorhanden sind. Die für das Aufbringen der Neuronzellen vorgesehenen Oberflächen 19 können dann z. B. auf Anschlußkontakten aufgebracht sein, die-dem in der Figur links eingezeichneten Meß-Anschlußkontakt 15 entsprechen und die über eine relativ dünne Kontaktlochfüllung entsprechend dem Anschluß 14 auf der linken Seite mit der Anschlußfläche 17 verbunden sind. Die Säulen 18 können statt aus Metall auch aus Silizium (Polysilizium oder amorphem Silizium) gebildet sein. Der Übersichtlichkeit halber nicht eingezeichnete weitere Details des erfindungsgemäßen Transistors entsprechen im wesentlichen den von im Rahmen eines CMOS-Prozesses hergestellten Transistoren bekannten Details.

## Patentansprüche

1. MOS-Transistor mit einer Gate-Elektrode (9) und mit einer Kontaktfläche (19) (Pad), die mit dieser Gate-Elektrode elektrisch leitend verbunden ist,
bei dem diese Kontaktfläche elektrochemisch korrosionsfest ist,
bei dem diese Kontaktfläche höchstens 200 nm höher oder tiefer angeordnet ist als eine die Kontaktfläche umgebende äußere Oberfläche (20) und
bei dem diese die Kontaktfläche umgebende Oberfläche mit einer passivierenden Deckschicht versehen ist.

2. MOS-Transistor nach Anspruch 1,
bei dem das Verhältnis von Gateweite zu Gatelänge größer als 3 ist.

3. MOS-Transistor nach Anspruch 1 oder 2,
bei dem das Verhältnis des Flächeninhalts der Kontaktfläche zu dem Flächeninhalt der Gate-Elektrode (Gateweite multipliziert mit Gatelänge) größer als 5 ist.

4. MOS-Transistor nach einem der Ansprüche 1 bis 3,
bei dem die Gate-Elektrode, die Kontaktfläche und die elektrisch leitende Verbindung dazwischen Polysilizium sind, das mit einem Metall aus der Gruppe von Titan, Tantal, Wolfram, Kobalt, Molybdän, Platin und Palladium siliziert ist.

5. MOS-Transistor nach einem der Ansprüche 1 bis 4,
bei dem die Gate-Elektrode, die Kontaktfläche und die elektrisch leitende Verbindung dazwischen mit einer Metallisierung versehen sind, die mindestens ein Material aus der Gruppe von Wolfram; Titan, Tantal, Kobalt, Molybdän, Platin, Iridium, Palladium und TiN umfaßt.

6. MOS-Transistor nach einem der Ansprüche 1 bis 5,
bei dem die Deckschicht ein Nitrid ist und eine Dicke von mindestens 50 nm und höchstens 250 nm besitzt.

## Claims

1. MOS transistor having a gate electrode (9) and having a contact area (19) (pad) which is electrically conductively connected to this gate electrode,
in which this contact area is electrochemically corrosion-resistant,
in which this contact area is arranged at most 200 nm higher or lower than an outer surface (20) surrounding the contact area, and
in which this surface surrounding the contact area is provided with a passivating covering layer.

2. MOS transistor according to Claim 1,
in which the ratio of gate width to gate length is greater than 3.

3. MOS transistor according to Claim 1 or 2,
in which the ratio of the surface area of the contact area to the surface area of the gate electrode (gate width multiplied by gate length) is greater than 5.

4. MOS transistor according to one of Claims 1 to 3,
in which the gate electrode, the contact area and the electrically conductive connection in between are polysilicon which is siliconized with a metal from the group consisting of titanium, tantalum, tungsten, cobalt, molybdenum, platinum and palladium.

5. MOS transistor according to one of Claims 1 to 4,
in which the gate electrode, the contact area and the electrically conductive connection in between are provided with a metallization layer which comprises at least one material from the group consisting of tungsten, titanium, tantalum, cobalt, molybdenum, platinum, iridium, palladium and TiN.

6. MOS transistor according to one of Claims 1 to 5,
in which the covering layer is a nitride and has a thickness of at least 50 nm and at most 250 nm.

## Revendications

1. Transistor MOS comprenant une électrode (9) de grille et une surface (19) de contact (Pad), qui est reliée de manière conductrice de l'électricité à cette électrode de grille,
dans lequel cette surface de contact est résistante à la corrosion par voie électrochimique,
dans lequel cette surface de contact et disposée au plus à 200 nm plus haut ou plus bas qu'une surface (20) extérieure entourant la surface de contact et
dans lequel cette surface entourant la surface de contact est munie d'une couche de finition passivante.

2. Transistor MOS suivant la revendication 1,
dans lequel le rapport de la largeur de la grille à la longueur de la grille est supérieur à 3.

3. Transistor MOS suivant la revendication 1 ou 2,
dans lequel le rapport de l'aire de la surface de contact à l'aire de l'électrode de grille (largeur de la grille multipliée par la longueur de la grille) est supérieur à 5.

4. Transistor MOS suivant l'une des revendications 1 à 3,
dans lequel l'électrode de grille, la surface de contact et la liaison conductrice de l'électricité entre elles sont en polysilicium qui est silicié par un métal choisi dans le groupe du titane, du tantale, du tungstène, du cobalt, du molybdène, du platine et du palladium.

5. Transistor MOS suivant l'une des revendications 1 à 4,
dans lequel l'électrode de grille, la surface de contact et la liaison conductrice de l'électricité entre elles sont munies d'une métallisation qui comprend au moins un matériau choisi dans le groupe du tungstène, du titane, du tantale, du cobalt, du molybdène, du platine, de l'iridium, du palladium et de TiN.

6. Transistor MOS suivant l'une des revendications 1 à 5,
dans lequel la couche de finition est un nitrure et a une épaisseur d'au moins 50 nm et d'au plus 250 nm.
